# EUROPÄISCHE PATENTANMELDUNG

(11) **EP 0 720 851 A2**
(43) Veröffentlichungstag der Anmeldung: **10.07.1996**
(21) Anmeldenummer: 95117773.2
(22) Anmeldetag: 11.11.1995
(51) Int. Cl.: A61K 31/47, A61K 31/535, A61K 31/54, A61K 31/14

(54) **Arzneimittel zur Inaktivierung von Viren mit Hilfe von Acridin oder Acridinderivaten**

(30) Priorität: 10.12.1994 DE 4444045
(71) Anmelder: BEHRINGWERKE Aktiengesellschaft, 35001 Marburg (DE)
(72) Erfinder: Bernhardt, Dieter, Dr., D-35091 Cölbe (DE)

(57) **Zusammenfassung**

Die Erfindung betrifft die Verwendung von Acridin oder Acridinderivaten, bevorzugt in Kombination mit Benzalkoniumchlorid, zur Inaktivierung von umhüllten oder nicht umhüllten Viren. Das erfindungsgemäße Verfahren wird bevorzugt in Gegenwart von Proteinen durchgeführt, deren biologische Aktivität dabei weitgehend erhalten bleibt.

## Beschreibung

Die Erfindung betrifft die Verwendung von Acridin oder Acridinderivaten, bevorzugt in Kombination mit Benzalkoniumchlorid, zur Inaktivierung von umhüllten oder nicht umhüllten Viren. Das erfindungsgemäße Verfahren wird bevorzugt in Gegenwart von Proteinen durchgeführt, deren biologische Aktivität dabei weitgehend erhalten bleibt.

Seit Jahren ist bekannt, daß unbehandeltes humanes Plasma oder Serum humanpathogene Viren enthalten kann, wie beispielsweise HIV, HBV oder HCV, die im Falle einer Übertragung auf empfängliche Rezipienten schwerwiegende Erkrankungen, wie AIDS oder Hepatitis verursachen können. Um diese potentielle Virusübertragung zu verhindern, werden Therapeutika, die aus humanem Plasma oder Serum gewonnen werden, zum einen nur aus vorselektierten, nach menschlichem Ermessen virusfreien Ausgangsmaterialien hergestellt und zum anderen virusinaktivierenden -/eliminierenden Schritten im Herstellungsverfahren unterworfen. Die Effizienz der angewandten Virusinaktivierungs -/eliminationsmethode wird dabei unter Anlegung strenger Maßstäbe nachgewiesen und ständig überprüft.

Neben physikalischen sind auch chemische Virusinaktivierungsschritte bei der Herstellung der genannten Therapeutika bekannt. Ein besonders häufig diskutiertes chemisches Verfahren ist die SD (solvent/detergent)-Methode. Sie ist dazu geeignet, umhüllte Viren, d. h. Viren, die von einer lipidhaltigen Membran umgeben sind, zu inaktivieren, hat jedoch den entscheidenden Nachteil, gegenüber allen bekannten nicht umhüllten (mantellosen) Viren völlig unwirksam zu sein. Darüber hinaus ist auch kein anderes chemisches Verfahren bekannt, welches geeignet wäre, nicht umhüllte Viren zu inaktivieren, bei gleichzeitigem Erhalt der biologischen Aktivität der Proteinbestandteile des Therapeutikums oder des humanen Plasmas oder Serums.

Obwohl die chemischen Virusinaktivierungsverfahren nur ergänzend zu den physikalischen Methoden angewandt werden, und obwohl die meisten potentiell durch Blut und Blutprodukte übertragbaren Viren einen Lipidmantel tragen, besteht aus Gründen der Sicherheit ein außerordentliches Bedürfnis, chemische Inaktivierungsverfahren bereitzustellen, die auch nicht umhüllte Viren zuverlässig inaktivieren. Dies wird umso mehr gewünscht, als kürzlich auch HAV und Parvoviren, wie beispielsweise Parvovirus B 19, als potentiell durch Blutflüssigkeiten oder Blutprodukte übertragbare Viren diskutiert wurden. (Vox Sanguinis 67, Supplement 1, 1994: Proceedings of a Symposium held at the New York Blood Center).

Der Erfindung lag also die Aufgabe zugrunde, ein industriell anwendbares Verfahren zur chemischen Virusinaktivierung zu entwickeln, wobei unter Erhalt der biologischen Aktivität anwesender, z. B. therapeutisch nützlicher Proteine, umhüllte und nicht umhüllte Viren, wie z. B. Parvoviren, inaktiviert werden.

Diese Aufgabe wird erfindungsgemäß dadurch gelöst, daß man Acridin oder ein Acridinderivat der zu behandelnden proteinhaltigen Flüssigkeit zusetzt. Es wurde überraschenderweise gefunden, daß Acridin oder Acridinderivate umhüllte und nicht umhüllte Viren inaktivieren. Acridinderivate sind beispielsweise Ethacridin, 9-Aminoacridin (=Aminacrin), 3,6-Acridindiamin (Proflavin) Acrisorcin, Acrizanchlorid (=Phenacridanchlorid), Acridinorange, Quinacrin, Acricid, Acridon, Acridin-9-carbonsäure, Acranil (1-[ (6-chloro-2-methoxy-9-acridinyl)-amino]-3-(diethylamin)-2-propanol dihydrochloride), 3,7-Diamino-5-phenyl-phenaziniumchlorid (Phenosafranin, Safranin B extra), Phenoxazin, Phenothiazin und v.a. Acriflavin (3,6 Diamino-10-methylacridiniumchlorid und 3,6-acridindiamin) und deren Salze wie z.B. Chloride, Sulfate, Bromide.

Überraschenderweise wurde außerdem gefunden, daß eine Kombination aus Acridin oder einem Acridinderivat und Benzalkoniumchlorid eine synergistische Wirkung bei der Virusinaktivierung entfaltet; d. h. die Größenordnung der Virusinaktivierung der Kombination liegt höher als die jeder Einzelsubstanz.

Das erfindungsgemäße Virusinaktivierungsverfahren kann mit Proteinlösungen wie Blut, Serum, Plasma, Blutprodukten, Allantoisflüssigkeit oder Milch durchgeführt werden. Die Virusinaktivierung wird bei einem pH von 3 bis 10 bzw. 5 bis 9 (bei z.B. Serum- oder Plasmalösungen) und einer Temperatur von 1°C bis 80°C, vorzugsweise von 20°C bis 60°C, ganz bevorzugt zwischen 20°C bis 40°C, bzw. 25°C bis 37°C durchgeführt und dauert 30 Minuten bis 10 Stunden, vorzugsweise 2 bis 5 Stunden. Zur Virusinaktivierung benutzt man für die Acridine bzw. Acridinderivate eine Konzentration von 1,0 g/l - 0,00001 g/l oder 1,0 g/l - 0,004 g/l, vorzugsweise 0,1 g/l - 0,001 g/l, für Benzalkoniumchlorid eine Konzentration von 0,1 g/l - 0,004 g/l, vorzugsweise 0,05 g/l - 0,01 g/l.

Die Entfernung der Acridine und des Benzalkoniumchlorids aus den Proteinlösungen, sofern dies notwendig ist, ist mittels einfacher, bekannter Methoden, wie Adsorption an Aktivkohle oder Dialyse möglich.

Ein weiterer Vorteil des erfindungsgemäßen Virusinaktivierungsverfahrens liegt in der weitestgehenden Schonung der Proteinbestandteile des zu behandelnden Materials: unterschiedliche biologische Aktivitäten, wie z. B. Antikörperaktivität und Gerinnungsaktivität, werden nicht oder nur in einem tolerierbaren Ausmaß reduziert.

Das erfindungsgemäße Virusinaktivierungsverfahren kann daher beispielsweise zur Dekontamination der folgenden Materialien eingesetzt werden:
- proteinhaltige Lösungen (verdünnt oder konzentriert)
- Blut oder Blutprodukte; sowohl die flüssigen als auch die zellulären Bestandteile
- Serum, Plasma
- Allantoisflüssigkeit
- Organextrakte
- Milch
- Pufferlösungen
- Antigene für Diagnostika
- Vaccine, Antigene für Vaccine
Weiterhin ist das erfindungsgemäße Verfahren zur Desinfektion bei Viruskontamination von beispielsweise Räumen, Geräten, Abwässern Abfällen und Oberflächen aller Art geeignet. Auch die Desinfektion von viruskontaminierten Organtransplantaten, wie z. B. Hornhaut, Hirnhäuten, Leber, Herz, Lunge oder Nieren ist möglich.

Die vorliegende Erfindung wird weiterhin durch die nachfolgenden Beispiele erläutert.

### Bei den erfindungsgemäßen Verfahren generell angewandte Methoden

Virus: Wurde in bekannter Weise in Gewebekulturen vermehrt, die Virusernten wurden zentrifugiert und dienten als Ausgangsstoff für die weiteren Untersuchungen. Der Infektiositätstiter wurde in Mikrotiterplatten - 8 Replica á 0.1 ml/ Verdünnungsstufe - durch Doppeltitration bestimmt.

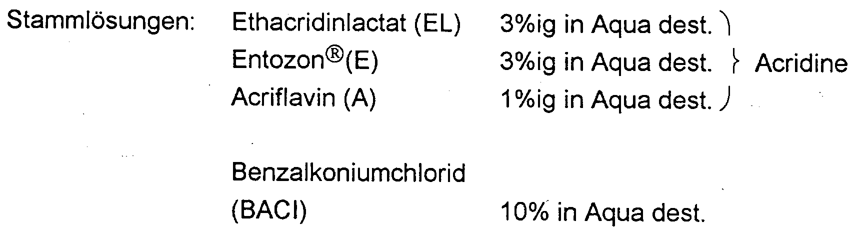

### Allgemeine Versuchsdurchführung:

9 Teile Puffer oder Medium oder Proteinlösungen wurden mit 1 Teil Virus gemischt. Danach erfolgte die in den Einzelbeispielen angegebene Mengenzugabe der Stammlösungen zur Virusinaktivierung und erneute Durchmischung mit anschließender Virustitration. Nach den in den Einzelbeispielen genannten Zeiten und Temperaturen wurden Proben entnommen und in Doppelbestimmungen titriert, um die verfahrensbedingte Virusinaktivierung messen zu können.

| **Untersuchte Virusarten** | |
|---|---|
| **Abkürzung** | **Name** |
| PI₃V | bovines Parainfluenza 3-Virus |
| BPV | bovines Parvovirus |
| PPV | porcines Parvovirus |
| IBRV | infektiöses bovines Rhinotracheitis-Virus |
| BVDV | bovines Virusdiarrhoevirus |
| CPV | canines Parvovirus |
| BAV-1 | bovines Adenovirus Typ 1 |
| Reo 3 | Reovirus Typ 3 |
| Influenza A | Influenza A-Virus Shangdong |
| Influenza B | Influenza B-Virus - B Panama |

Im Text verwendete weitere Abkürzungen/Bezeichnungen:
- EME-Medium: Eagles Minimum Essential Medium
- Beriate ® P: Gerinnungsfaktor VIII:C-Konzentrat, pasteurisiert (Behringwerke AG, Marburg, Deutschland)
- Haemate ® P: Konzentrat aus Gerinnungsfaktor VIII und Von-Willebrand-Faktor, pasteurisiert (Behringwerke AG, Marburg, Deutschland)
- Beriplex ® P: pasteurisiertes Prothrombin-Komplex-Konzentrat (Behringwerke AG, Marburg, Deutschland)
- Venimmun ®: humane polyvalente Immunglobulin-Präparation (7S) zur intravenösen Anwendung (Behringwerke AG, Marburg, Deutschland)
- Entozon ®: Präparat folgender Zusammensetzung: 1 g Granulat enthält 0,059 g Dimethoxy-6-nitro-9-[(3-diethylamino-2-hydroxy)-propylamino]-acridindihydrochlorid und 0,295 g Ethacridinlactat (ASID Veterinär Vertriebs GmbH)
- QAE-Cellulose: Diethyl-2-hydroxypropylaminoethyl-Cellulose (Ionenaustauscher zur Proteinreinigung)
- FKS: fötales Kälberserum

### Beispiel 1

### Inaktivierung von PI₃V durch BACI

EME-Medium wurde mit PI₃-Virus und BACI gemischt und bei 37°C im Wasserbad inkubiert. Nach den angegebenen Zeiten wurden Proben zur Prüfung der Virusinaktivierung entnommen. Die Ergebnisse sind in Tab. 1 aufgeführt.

**Tabelle 1**

| **Inaktivierung von PI**_{**3**}**-Virus mit BACI bei 37°C** | | | |
|---|---|---|---|
| _Zeit (h) | BACI Zugabe (mg/ml) | festgestellter Virustiter (log₁₀/ml | Virusinaktivierung (log₁₀/ml) |
| 0-0,03¹⁾ | 0 (Kontrolle) | 7,4 | - |
| | 0,1 | ≦1,5 | ≧5,9 |
| | 0,05 | ≦1,5 | ≧5,9 |
| | 0,025 | 6,0 | 1,4 |
| | 0,0125 | 6,9 | 0,5 |
| 1 | 0 (Kontrolle) | 6,8 | - |
| | 0,1 | ≦1,5 | ≧5,3 |
| | 0,05 | ≦1,5 | ≧5,3 |
| | 0,025 | ≦1,5 | ≧5,3 |
| | 0,0125 | 5,5 | 1,3 |

| | | | |
|---|---|---|---|
| ¹⁾ gleich nach Zugabe von BACI und Durchmischen des Reaktionsansatzes | | | |

Wie aus den Resultaten in Tabelle 1 hervorgeht, wird PI₃-Virus durch die hohen Dosen BACI (0,1 mg/ml, 0,05 mg/ml) sehr rasch inaktiviert, d.h. in der Zeit, die benötigt wurde, die virushaltige Probe mit BACI gut zu durchmischen, eine Probe zu entnehmen und diese zu titrieren, um die Infektiosität von PI₃-Virus festzustellen, war kein infektiöses Virus mehr nachweisbar. Bei den beiden anderen geprüften BACI-Konzentrationen (0,025 mg/ml und 0,0125 mg/ml) ist eine klare Konzentrations-Zeitabhängigkeit der Virusinaktivierung erkennbar.

### Beispiel 2

### Virusinaktivierung mittels BACI oder Entozon

Die in Tab. 2 aufgeführten Virusarten wurden mit BACI oder Entozon versetzt und bei 45°C inkubiert. Die Probenentnahme für die Virustitration erfolgte 1 oder 2 Stunden nach Testbeginn.

**Tabelle 2**

| **Virusinaktivierung mittels BACI oder Entozon bei 45 °C** | | | | |
|---|---|---|---|---|
| Virus | Zeit | Substanzzugabe (mg/ml) | festgestellter Virustiter (log₁₀/ml) | Virusinaktivierung (log₁₀/ml) |
| BPV | 1 Stunde | Kontrolle 0 | 4,7 | 0 |
| | | BACI 0,05 | 5,2 | 0 |
| | | BACI 0,01 | 5,2 | 0 |
| | 2 Stunden | Kontrolle 0 | 4,6 | 0 |
| | | BACI 0,05 | 4,6 | 0 |
| | | BACI 0,01 | 4,6 | 0 |
| | 1 Stunde | Kontrolle 0 | 4,7 | 0 |
| | | Entozon 0,030 | ≦1,5 | ≧3,2 |
| | | Entozon 0,015 | ≦1,5 | ≧3,2 |
| | 2 Stunden | Kontrolle 0 | 4,6 | 0 |
| | | Entozon 0,030 | ≦1,5 | ≧3,1 |
| | | Entozon 0,015 | ≦1,5 | ≧3,1 |
| PPV | 1 Stunde | Kontrolle 0 | 5,6 | 0 |
| | | Entozon 0,030 | 3,3 | 2,3 |
| | | Entozon 0,015 | 3,7 | 1,9 |
| | 2 Stunden | Kontrolle 0 | 5,6 | 0 |
| | | Entozon 0,030 | 2,2 | 3,4 |
| | | Entozon 0,015 | 2,7 | 2,9 |

Wie die Ergebnisse in Tabelle 2 zeigen, wird BPV durch BACI unter den gewählten Versuchsbedingungen nicht inaktiviert. Mittels Entozon ist eine Inaktivierung möglich, wobei die Inaktivierung beim BPV wesentlich schneller als beim PPV erfolgt.

### Beispiel 3

### Virusinaktivierung mittels BACI und Acriflavin

Die in Tab. 3 aufgeführten Suspensionen der Virusarten wurden mit BACI oder Acriflavin versetzt und bei 37 °C 2 Stunden inkubiert. Danach wurden Proben für die Titration zur Feststellung der Virusinaktivierung entnommen.

**Tabelle 3**

| Virus | Substanzzugabe (mg/ml) | festgestellter Virustiter (log₁₀/ml) | Virusinaktivierung (log₁₀/ml) |
|---|---|---|---|
| IBRV | Kontrolle 0 | 5,1 | 0 |
| | BACI 0,01 | ≦1,5 | ≧3,6 |
| | Acriflavin 0,001 | 1,8 | 3,3 |
| PI₃V | Kontrolle 0 | 5,6 | 0 |
| | BACI 0,01 | ≦1,5 | ≧4,1 |
| | Acriflavin 0,001 | 3,2 | 2,4 |
| BVDV | Kontrolle 0 | 6,2 | 0 |
| | BACI 0,01 | 2,3 | 3,9 |
| | Acriflavin 0,001 | 2,5 | 3,7 |
| BPV | Kontrolle 0 | 5,4 | 0 |
| | BACI 0,01 | 5,9 | 0 |
| | Acriflavin 0,001 | ≦1,5 | ≧3,9 |

Wie die Ergebnisse in Tabelle 3 zeigen, werden die umhüllten Virusarten - IBRV, PI₃V, BVDV - sowohl durch BACI und Acriflavin inaktiviert, wobei die Inaktivierung durch BACI etwas stärker ist. Das nackte Virus BPV wird durch Acriflavin inaktiviert, nicht jedoch durch BACI alleine.

Nachdem die Virusinaktivierung mittels Acriflavin und BACI in EME-Medium nachgewiesen war, überprüften wir, ob diese Substanzen auch Virus in proteinhaltigen Lösungen inaktivieren können. Die folgenden Proteinlösungen wurden mit den angegebenen Virusarten versetzt:
- Beriplex ®: PPV
- Pferdeserum: BVDV, BPV
- Beriate ®: BVDV, BPV, BAV-1, Reo 3, IBRV
- Haemate ®: PPV, Reo 3
- Venimmun ®: BVDV, PPV, CPV
- FKS: CPV
- Ei-Allantoisflüssigkeit: Influenza A, Influenza B
Die bei diesen Versuchen erzielten Resultate sind nachfolgend tabellarisch aufgeführt.

### Beispiel 4

### Virusinaktivierung mit Acridinen und Benzalkoniumchlorid in Proteinlösungen

Wie aus den Ergebnissen in Tabelle 4 hervorgeht, ist es möglich, ganz unterschiedliche Viren mit Acridinen und/oder Benzalkoniumchlorid, methodisch einfach, in Proteinlösungen zu inaktivieren. Hierbei zeigt sich aber klar, daß mittels Benzalkoniumchlorid alleine nur hüllhaltige Viren zu inaktivieren sind, während mittels Acridinen sowohl hüllhaltige, als auch hüllenlose Virusarten inaktiviert werden. Beide Substanzen wirken in ihrer viruziden Wirkung synergistisch, d.h. die Größenordnung der Virusinaktivierung ist bei der Kombination Acriflavin + Benzalkoniumchlorid höher als bei den beiden Einzelsubstanzen.

Die Resultate in Tabelle 4 zeigen auch, daß die Virusinaktivierung in Proteinlösungen abhängig ist von:
1. der zu inaktivierenden Virusart
2. den Bestandteilen und Art der Proteinlösung
3. der Inaktivierungsmittelkonzentration
4. der Inaktivierungszeit
5. der Inaktivierungstemperatur.

Ferner ist die Virusinaktivierung pH-abhängig - Resultate nicht gezeigt. Bei pH unter 5,5 verläuft die Virusinaktivierung langsamer als bei höheren pH-Werten.

Die Tabellen 5 - 7 beinhalten die Resultate der biologischen Wirksamkeit der Proteinlösungen nach Virusinaktivierung mittels Acriflavin und/oder Benzalkoniumchlorid. Die Bestimmung der biologischen Wirksamkeit erfolgte beim Venimmun ® durch den Gehalt an Antikörpern vor und nach Virusinaktivierung, bei Haemate ®, Beriate ® und Beriplex ® durch die Bestimmung der gerinnungsfördernden Aktivität - gemessen in internationalen Einheiten.

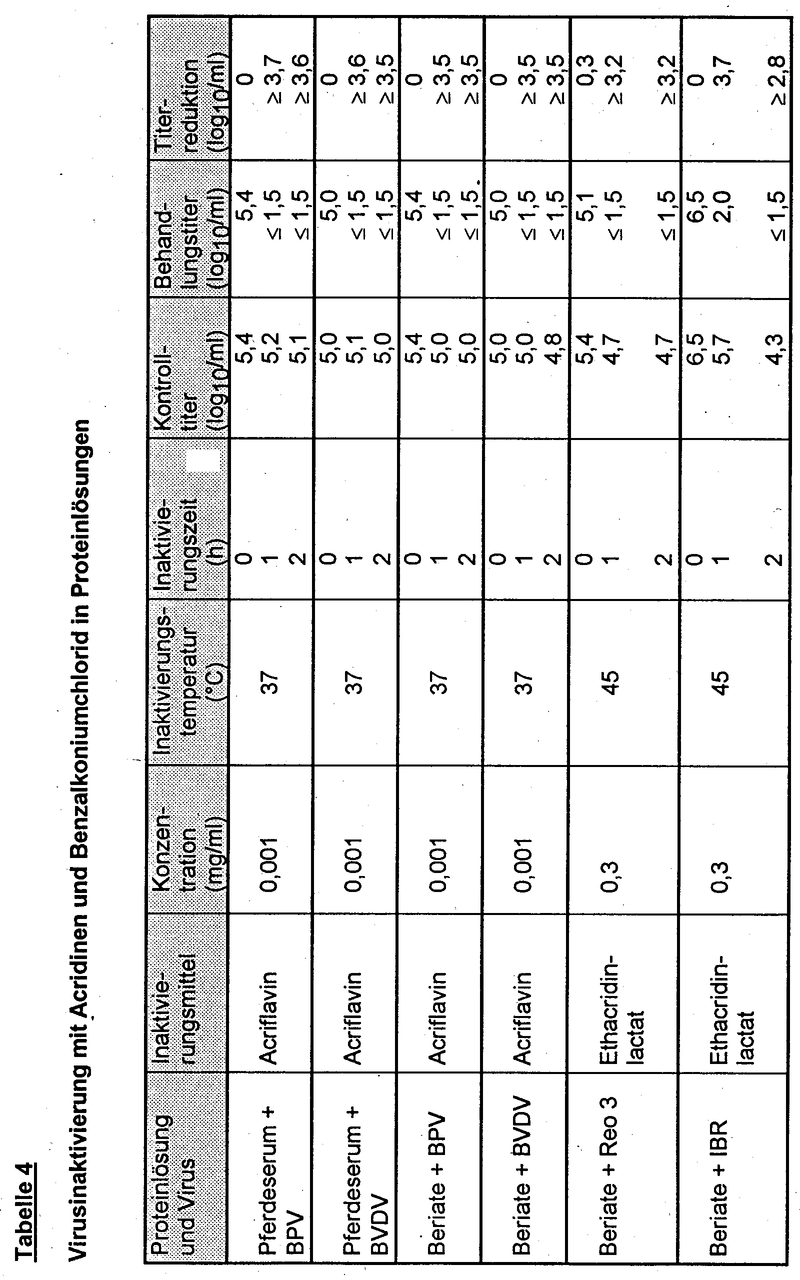

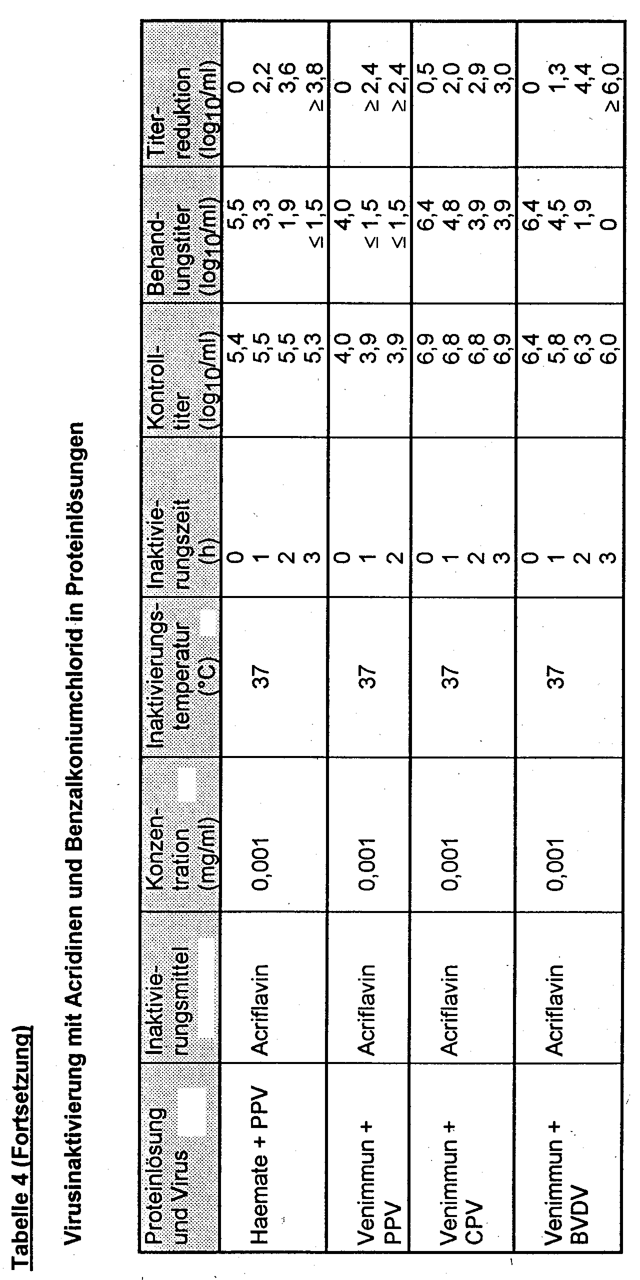

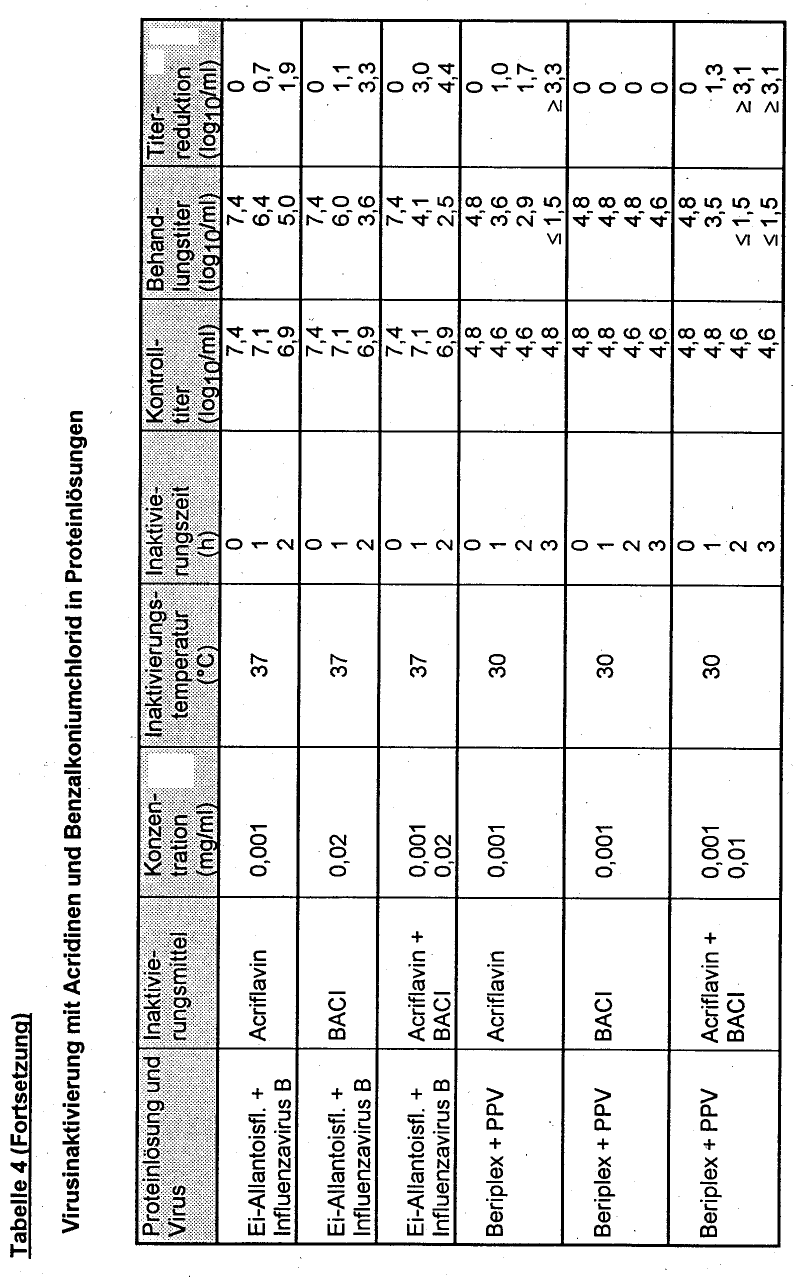

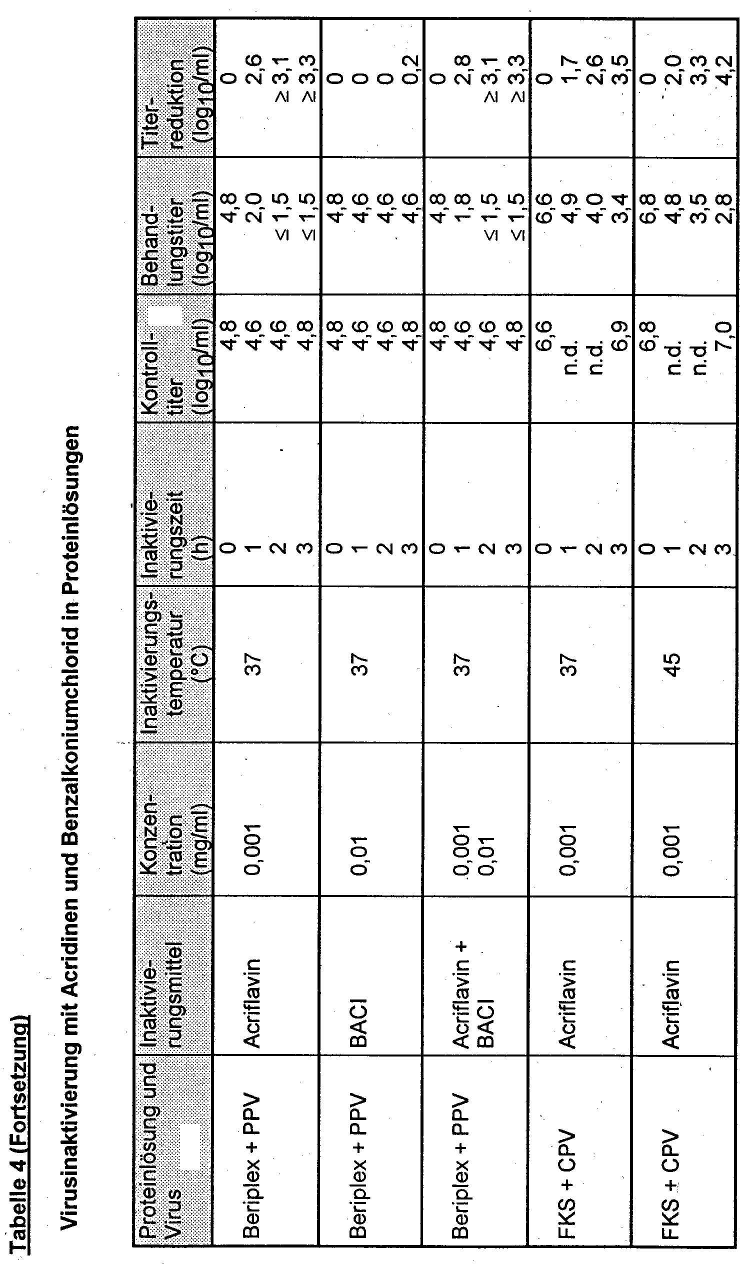

**Tabelle 5**

| **Bestimmung der biologischen Aktivität (Antikörpertiter) in Proteinlösungen vor und nach Virusinaktivierung mittels Acriflavin und Benzalkoniumchlorid** | | | | |
|---|---|---|---|---|
| Proteinlösung | Bezeichnung | Temperatur u. Dauer der Behandlung | rezipr. Neutralisationstiter ① gegen Poliovirus Typ 1 | |
| | | | ohne Inaktivierungs -mittel | mit Inaktivierungs -mittel ② |
| kryoarmes humanes Plasma (Einzelplasma) | H1 | 37°C 2 Stunden | 646 | 646 |
| | H2 | | 741 | 562 |
| | H3 | | 741 | 646 |
| | H4 | | 646 | 741 |
| | H5 | | 376 | 562 |
| Venimmun ® (Endprodukt) verschiedene Chargen | V1 | 37°C 2 Stunden | 2234 | 851 |
| | V2 | | 1950 | 1698 |
| | V3 | | 1698 | 1698 |
| | V4 | | 1698 | 1698 |
| | V5 | | 1479 | 1479 |
| kryoarmes humanes Plasma (Einzelplasma) | H1 | 45°C 2 Stunden | 427 | 977 |
| | H2 | | 1288 | 977 |
| | H3 | | 1288 | 977 |
| | H4 | | 977 | 1122 |
| | H5 | | 977 | 1288 |
| Venimmun ® (Endprodukt) verschiedene Chargen | V1 | 45°C 2 Stunden | 1950 | 2234 |
| | V2 | | 2570 | 2234 |
| | V3 | | 2234 | 1479 |
| | V4 | | 2234 | 1950 |
| | V5 | | 2234 | 1479 |

| | | | | |
|---|---|---|---|---|
| ① Titerberechnung nach Spearman-Kärber aus log₂-Verdünnungsreihe mit 8 Replicate/Verdünnungsstufe | | | | |
| ② Acriflavin 0,001 mg/ml + BACI 0,02 mg/ml | | | | |

**Tabelle 6**

| **Bestimmung der biologischen Aktivität (Antikörpertiter) in Proteinlösungen vor und nach Virusinaktivierung mittels Acriflavin und Benzalkoniumchlorid** | | | | | | |
|---|---|---|---|---|---|---|
| Proteinlösung | Bezeichnung | Temperatur u. Dauer der Behandlung | rez. HAH-Anti-PI₃-Virus Antik.-Titer ① | | rez. HAH-Anti-Reo3-Virus Antik.-Titer ① | |
| | | | ohne I. ② | mit I. ② | ohne I. ② | mit I. ② |
| kryoarmes humanes Plasma (Einzelplasma) | H1 | 45°C 2 Stunden | 80 | 80 | 320 | 320 |
| | H2 | | 80 | 80 | 320 | 320 |
| | H3 | | 80 | 80 | 320 | 320 |
| | H4 | | 80 | 80 | 320 | 320 |
| | H5 | | 80 | 80 | 320 | 320 |
| Venimmun ® (Endprodukt) verschiedene Chargen | V1 | 45°C 2 Stunden | 320 | 320 | 320 | 320 |
| | V2 | | 320 | 320 | 320 | 320 |
| | V3 | | 320 | 320 | 320 | 320 |
| | V4 | | 320 | 320 | 320 | 320 |
| | V5 | | 320 | 320 | 320 | 320 |
| Pferdeserum (Einzelseren) | P1 | 45°C 2 Stunden | n.d. | n.d. | 640 | 640 |
| | P2 | | | | 640 | 640 |
| | P3 | | | | 640 | 640 |
| | P4 | | | | 640 | 640 |
| | P5 | | | | 640 | 640 |

| | | | | | | |
|---|---|---|---|---|---|---|
| ① Titerbestimmung in log₂-Verdünnungsreihe | | | | | | |
| ② I. = Inaktivierungsmittel: Acriflavin 0,001 mg/ml + BACI 0,02 mg/ml | | | | | | |

**Tabelle 7**

| **Bestimmung der biologischen Aktivität (Gerinnungsaktivität) von Proteinlösungen vor und nach Virusinaktivierung mittels Acriflavin (A) und Benzalkoniumchlorid (BaCI)** | | | | |
|---|---|---|---|---|
| Proteinlösung | Temperatur u. Dauer der Behandlung | Inaktivierungsmittel | Inaktivierungsmittelkonzentration (mg/ml) | Aktivität (IU/ml) |
| Kryoproteinlösung nach Al(OH)₃ -+ QAE -Behandlung | 3 Stunden 45°C | A | 0,01 | 3,9 |
| | | A | 0,03 | 4,2 |
| | | A | 0,001 | 4,4 |
| | | BACI | 0,1 | 6,9 |
| | | BACI | 0,05 | 6,1 |
| | | A + BACI | 0,001 + 0,05 | 4,1 |
| | | Kontrolle | 0 | 5,3 |
| Haemate ® FaktorVIII | 3 Stunden 37°C | A | 0,002 | 18,9 |
| | | A | 0,001 | 16,8 |
| | | BACI | 0,02 | 20,7 |
| | | A + BACI | 0,001 + 0,02 | 17,6 |
| | | Kontrolle | 0 | 24,1 |
| Beriplex ® Faktor II | 3 Stunden 37°C | A | 0,002 | 48,8 |
| | | A | 0,001 | 50,0 |
| | | BACI | 0,02 | 61,3 |
| | | A + BACI | 0,001 + 0,02 | 47,9 |
| | | Kontrolle | 0 | 68,6 |

Wie aus den Resultaten in Tabelle 5 und 6 hervorgeht, wird der Gehalt an neutralisierenden (Polio Typ 1) und hämagglutinationshemmenden (HAH) Antikörpern (PI₃-, Reo 3 Virus) in Venimmun ® bei der Virusinaktivierung mittels Acridinen und Benzalkoniumchlorid nicht über die Testschwankungen (in der Regel ± 1 log₂-Stufe) hinausgehend, beeinflußt. Bei den Gerinnungspräparaten (Beriate ®, Haemate ®, Beriplex ®) ist der Aktivitätsabfall nach Acridinen/ Benzalkoniumchlorid Inaktivierung tolerierbar (Tabelle 7).

## Patentansprüche

1. Verfahren zur Inaktivierung von Viren, dadurch gekennzeichnet, daß Acridin oder ein Acridinderivat eingesetzt wird.

2. Verfahren nach Anspruch 1, dadurch gekennzeichnet, daß zusätzlich Benzalkoniumchlorid eingesetzt wird.

3. Verfahren nach Anspruch 1 oder 2, dadurch gekennzeichnet, daß es in Anwesenheit von Proteinen durchgeführt wird und die biologische Aktivität dieser Proteine erhalten bleibt.

4. Verfahren nach Anspruch 1, 2 oder 3, dadurch gekennzeichnet, daß die Inkubation bei einer Temperatur von 20-60 °C durchgeführt wird.

5. Verfahren nach einem der vorhergehenden Ansprüche 1 - 4, dadurch, gekennzeichnet, daß die Temperatur 25-37 °C beträgt.

6. Verfahren nach einem der vorhergehenden Ansprüche 1 - 5, dadurch gekennzeichnet, daß die Inkubation bei pH 5-9 durchgeführt wird.

7. Verfahren nach einem der vorhergehenden Ansprüche 1 - 6, dadurch gekennzeichnet, daß Acridin oder das Acridinderivat in einer Konzentration von 0,0001 bis 1,0 g/l eingesetzt wird.

8. Verfahren nach Anspruch 7, dadurch, gekennzeichnet, daß Acridin oder das Acridinderivat in einer Konzentration von 0,0005 bis 0,1 g/l eingesetzt wird.

9. Verfahren nach einem der vorhergehenden Ansprüche 2 - 8, dadurch gekennzeichnet, daß Benzalkoniumchlorid in einer, Konzentration von 0,004 bis 0,1 g/l eingesetzt wird.

10. Verfahren nach Anspruch 9, dadurch gekennzeichnet, daß Benzalkoniumchlorid in einer Konzentration von 0,001 bis 0,05 g/l eingesetzt wird.

11. Verfahren nach einem der vorhergehenden Ansprüche 1 - 10, dadurch gekennzeichnet, daß die Inkubationszeit 0,5 h - 10,0 h beträgt.

12. Verfahren nach Anspruch 11, dadurch gekennzeichnet, daß die Inkubationszeit 2-5 h beträgt.

13. Verfahren nach einem der vorhergehenden Ansprüche 1 - 12, dadurch gekennzeichnet, daß die Viren Parvoviren oder andere unbehüllte Viren sind.

14. Verfahren nach einem der vorhergehenden Ansprüche 1 - 13, dadurch gekennzeichnet, daß die Viren umhüllte Viren sind.
